(19) Europäisches Patentamt
European Patent Office
Office européen des brevets

(11) Publication number: **0 066 301 B1**

(12) **EUROPEAN PATENT SPECIFICATION**

(45) Date of publication of patent specification: **22.01.86**

(51) Int. Cl.$^4$: **C 07 D 205/08,** C 07 F 7/10 // C07D487/04, C07C149/437

(21) Application number: **82105683.5**

(22) Date of filing: **01.05.79**

(60) Publication number of the earlier application in accordance with Art. 76 EPC: **0 007 973**

(54) **Intermediates for the preparation of thienamycin and process for preparing the same.**

(30) Priority: **14.08.78 US 933323**

(43) Date of publication of application: **08.12.82 Bulletin 82/49**

(45) Publication of the grant of the patent: **22.01.86 Bulletin 86/04**

(84) Designated Contracting States: **AT BE CH DE FR GB IT LU NL SE**

(56) References cited:
**US-A-4 122 086**

**TETRAHEDRON LETTERS, no. 14, April 1974, pages 1271-1274, Pergamon Press, (GB); K. CLAUSS: "$-Chlormethyl-4-Methyl-Azetidin-2-on"**

(73) Proprietor: **MERCK & CO. INC.
126, East Lincoln Avenue P.O. Box 2000
Rahway New Jersey 07065 (US)**

(72) Inventor: **Christensen, Burton Grant
195 Watchung Terrace
Scotch Plains, N.J. 07060 (US)**
Inventor: **Salzmann, Thomas Norse
387 Brook Avenue
North Plainfield, N.J. 07062 (US)**
Inventor: **Ratcliffe, Ronald William
234 Matawan Avenue
Matawan, N.J. 07747 (US)**

(74) Representative: **Abitz, Walter, Dr.-Ing. et al
Abitz, Morf, Gritschneder, Freiherr von Wittgenstein Postfach 86 01 09
D-8000 München 86 (DE)**

**EP 0 066 301 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to compounds of the formula

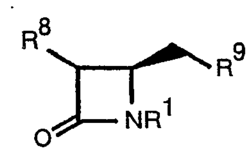

wherein

a) $R^1$ is hydrogen or a protecting group,
$R^8$ is hydrogen and
$R^9$ is iodo,

b) $R^1$ is a protecting group,
$R^8$ is

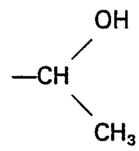

$R^9$ and is $—C(SR)_3$
wherein R is selected from alkyl having 1—6 carbon atoms, aryl and aralkyl having 6 to 10 carbon atoms, and wherein R can be the same or different,

c) $R^1$ is hydrogen,
$R^8$ is

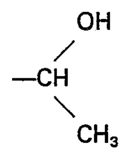

and
$R^9$ is

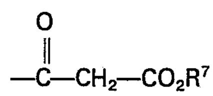

wherein $R^7$ is a protecting group.

d) $R^1$ is hydrogen,
$R^8$ is

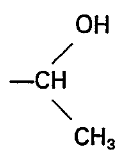

and
$R^9$ is

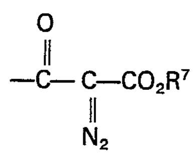

wherein $R^7$ is a protecting group.

These compounds can be used as intermediates for the total synthesis of the known antibiotic thienamycin (I)

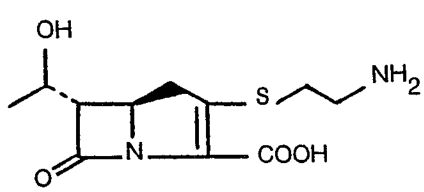

I

Starting from L-aspartic acid, the synthesis proceeds in a stereo-selective way *via* intermediates II, III, IV:

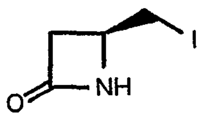
II
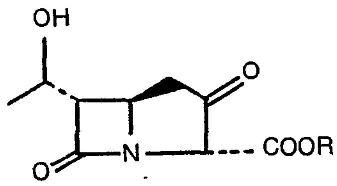
III
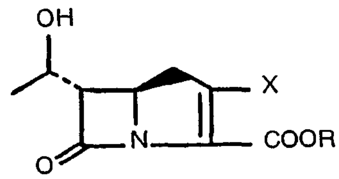
IV

wherein X is a conventional leaving group, such as halo or organosulfonyloxy, and R is hydrogen, a conventional, readily removable protecting group, such as an ester, or a salt cation. The details of the total synthesis are given below.

The intermediates of the present invention may conveniently be prepared and used in the process which is summarized by the following reaction diagram:

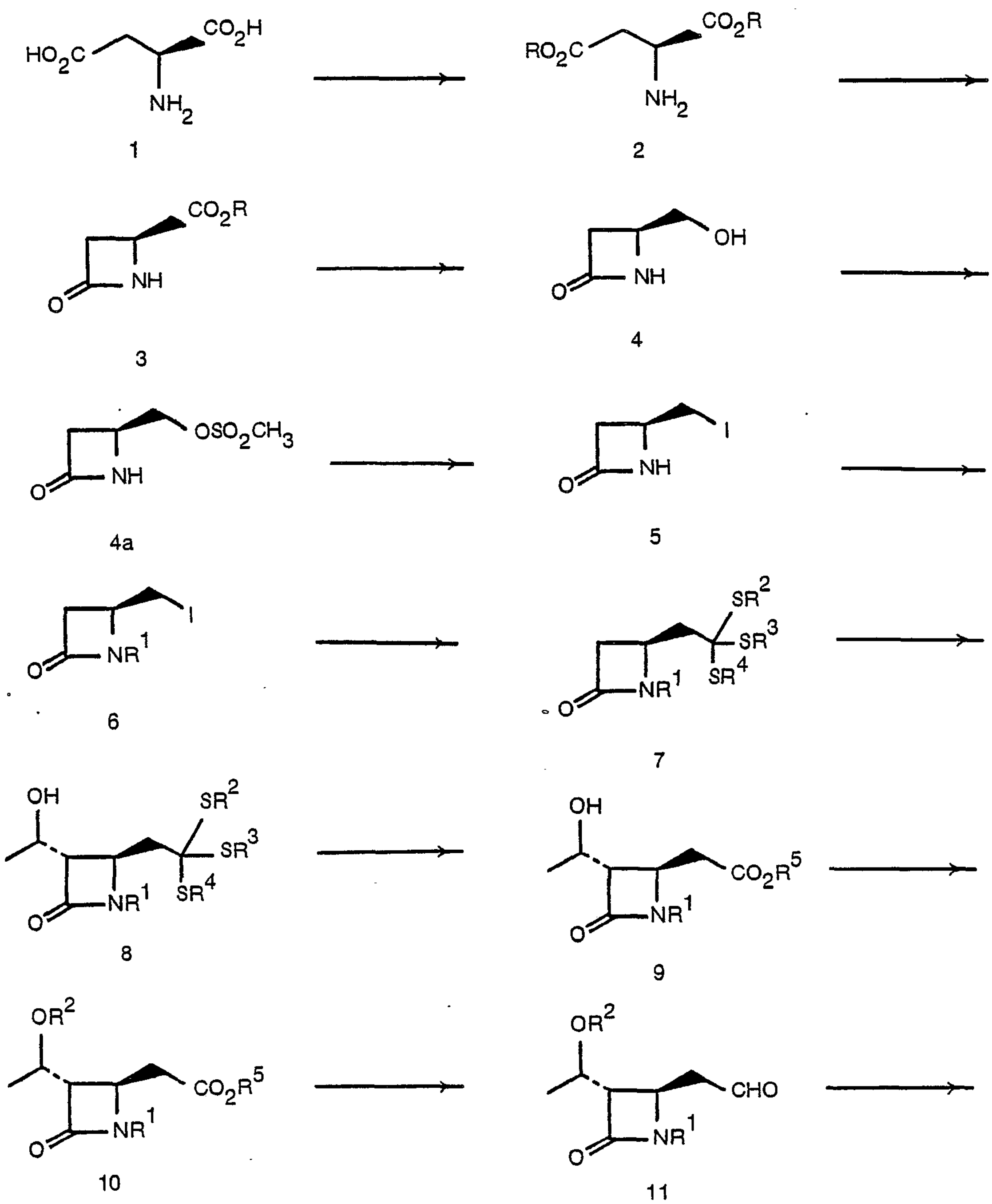
1 2 3 4 4a 5 6 7 8 9 10 11

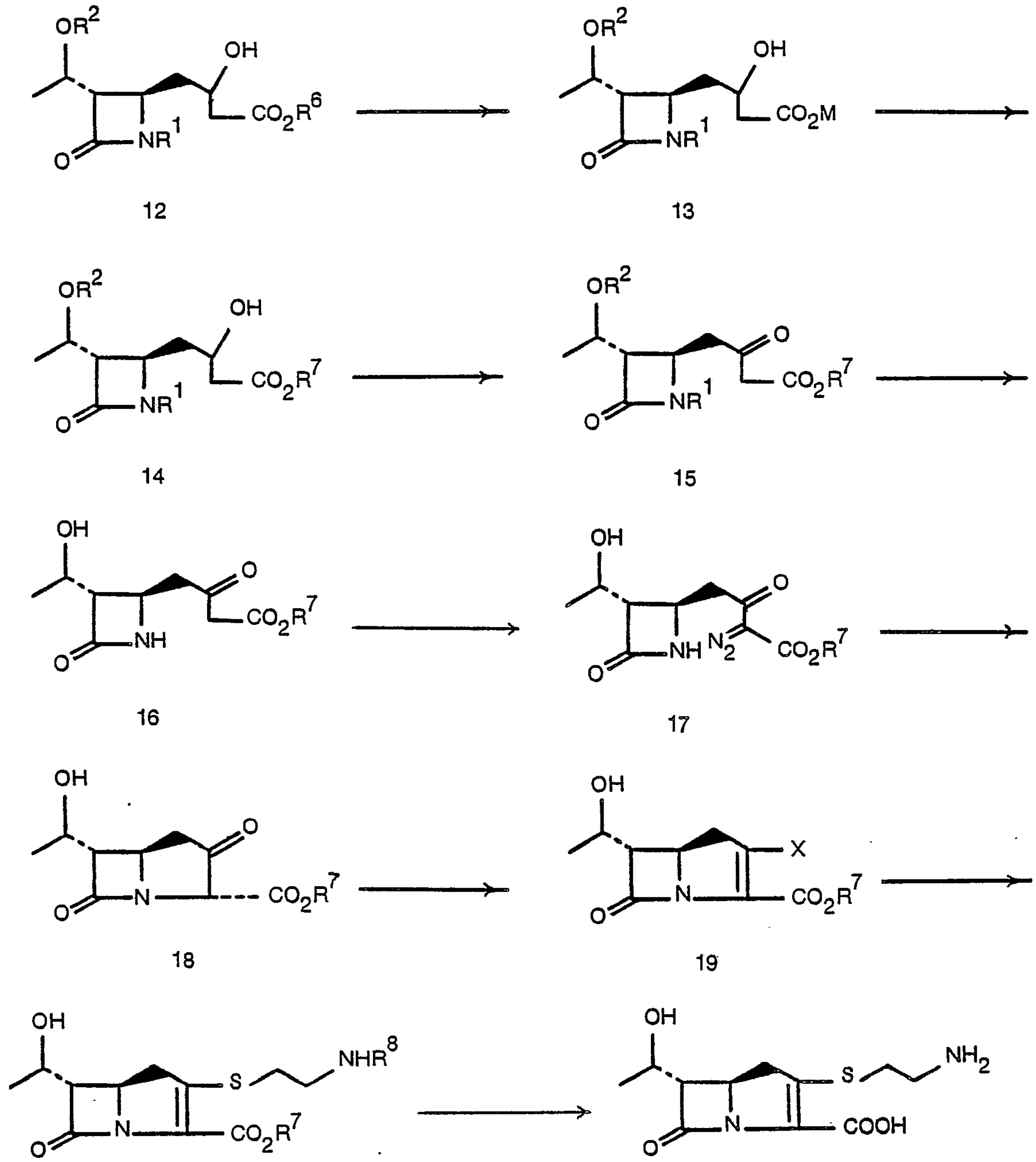

12 13

14 15

16 17

18 19

20 I

In words relative to the above diagram, L-aspartic acid 1 is esterified according to well known procedures. Typically 1 in a solvent such as benzene, toluene or chloroform is treated with an esterifying agent such as benzyl alcohol, methanol, ethanol or isopropanol, in the presence of for example p-toluene sulfonic acid, HCl, HBr, at a temperature of from 0 to 110°C for from 1 to 24 hours to achieve the desired establishment and hence protection of the carboxyl functions. The resulting species 2 in a solvent such as ether, THF or DME is treated with for example trimethylchlorosilane, followed by treatment with for example EtMgBr, MeMgI, ØMgBr or t-BuMgCl, at a temperature of form −40 to 50°C for from 1 to 72 hours to provide azetidinone 3. Reduction of species 3 with a reducing agent such as $NaBH_4$, in a solvent such as methanol, ethanol or isopropanol at a temperature of from −10 to 40°C for from 1 to 6 hours provides 4. (For purposes here, the symbols: Et, Me, Ø, iPr, and t-Bu stand for: ethyl, methyl, phenyl isopropyl, and tert-butyl, respectively.)

Treatment of 4 in such a solvent such as methylene chloride or $CHCl_3$ with for example methane sulfonyl chloride or methane sulfonic anhydride in the presence of a base such as $Et_3N$ or $Ipr_2NEt$, followed by treatment with a stoichiometric to 5 fold exccxess of sodium iodide in acetone yields 5 *via* 4a.

The transformation 5→6 establishes the protecting group $R^1$ which may be a triorganosilyl group, such

as t-butyldimethylsilyl, t-butyldiphenylsilyl, triphenylsilyl, isopropyldimethylsilyl, for example, or may be 3,4-dimethoxybenzyl, for example. Silyl protection is preferred, and typically $R^1$ is established by treating 5 in a solvent such s dimethylformamide, acetonitril, hexamethylphosphoramide or tetrahydrofuran with a silylating agent such as t-butyldimethylchlorosilane, t-butyldiphenylchlorosilane and triphenylchlorosilane, at a temperature of from −20° to 25°C for from 0.5 to 24 hours in the presence of a base such as triethylamine, diisopropylmethylamine, or imidazole.

The transformation 6→7 is accomplished by treating 6 in a solvent such as tetrahydrofuran, dimethoxyethane or diethylether with a carbanion generically represented by the following structure:

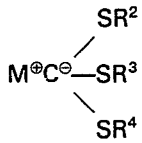

wherein M is a metal cation such as lithium, potassium, copper or magnesium, for example, and $R^2$, $R^3$ and $R^4$ are selected from alkyl, especially having 1 to 6 carbon atoms, aryl or aralkyl, especially having 6 to 10 carbon atoms, such as methyl, ethyl, benzyl, methoxybenzyl, trityl and phenyl, for exasmple, at a temperature of from −100 to 0°C and from 0.5 to 4 hours. Typically, the carbanion reagent is prepared prior to addition of substrate 6 on treatment of the triorganothiomethane with a strong base such as n-butyllithium, t-butyllithium, phenyllithium, or lithium diisopropylamide (LDA).

The alkylation 7→8 is accomplished by treating 7 in a solvent such as tetrahydrofuran, dimethoxyethane, diethylether, hexamethylphosphoramide, at a temperature of from −100° to −20°C with a strong base such as lithium diisopropylamide, lithium hexamethyldisilazide, lithium 2,2,6,6-tetramethylpiperidide or potassium hydride followed by the addition of an equivalent to 10 fold excess of acetaldehyde. This reaction gives a mixture of isomers from which the desired *trans*-R form can be conveniently separated by chromatography or crystallization.

The transformation 8→9 is accomplished by treating 8 in a solvent such as methanol, ethanol or isopropanol at a temperature of form 0 to 80°C with a Lewis acid such as mercuric chloride, silver tetrafluoroborate or thallium trinitrate. The value of $R^5$ is determined by the identity of the alcohol taken in reaction.

The transformation 9→10 establishes the hydroxyl protecting group $R^2$. The most preferred protecting groups $R^2$ are triorganosilyl groups such as t-butyldimethylsilyl, t-butyldiphenylsilyl and triphenylsilyl. Typically, silylation is accomplished by treating 2 with the corresponding triorganosilyl chloride in a solvent such as dimethylformamide, acetonitrile and tetrahydrofuran at a temperature of from −20 to 80°C for from 0.5 to 24 hours.

The reduction 10→11 is accomplished by treating 10 in a solvent such as toluene, methylene chloride diethylether and tetrahydrofuran with a reducing agent such as diisobutylaluminum hydride, sodium bis(2-methoxyethoxy)aluminum hydride a temperature of from −100° to −40°C for from 1 to 10 hours.

The addition 11→12 is accomplished by treating 11 in a solvent such as tetrahydrofuran, diethylether, dimethoxyethane at a temperature of from −100° to 0°C for from 15 minutes to 2 hours in the presence of $LiCH_2CO_2R^6$; wherein $R^6$ is benzyl, p-methoxybenzyl, 2,4-dimethoxybenzyl for example; which reagent is typically generated *in situ* on treatment of the appropriate $R^6$ acetate with a strong base such as LDA, lithium hexamethyldisilazide or lithium 2,2,6,6-tetramethylpiperide.

If desired, a more readily removable carboxyl protecting group may conveniently replace the first established group, $R^6$, by the carboxyl protecting group $R^7$. This transformation 12→13→14 is accomplished by selectively deblocking 12 to form 13 by hydrogenation or hydrolysis. Typically, the reaction is accomplished by treating 12 in a solvent such as methanol, ethanol, dioxane, tetrahydrofuran or water under a hydrogenation pressure of from 1 to 4 atmospheres in the presence of a catalyst such as Pd on charcoal or $Pd(OH)_2$, for from 0.1 to 10 hours. The 13 intermediate (M may be H, Na, K or ammonium such as $Et_3NH$, for example) need not be isolated. Intermediate 14 is obtained from the hydrogenation mixture upon treatment with the chosen reagent calculated to establish the protecting group $R^7$ such as an aralkyl halide in a solvent such as dimethylformamide, acetonitrile, hexamethylphosphoramide at a temperature of from 0° to 50°C for from 18 hours. $R^7$ is typically an aralkyl group such as p-nitrobenzyl, or o-nitrobenzyl, for example.

The oxidation 14→15 is accomplished by treating 14 in a solvent such as methylene chloride or acetonitrile, with an oxidizing system such as dipyridine chromium (VI) oxide, 3,5-dimethylpyrazole chromium (VI) oxide, pyridinium chlorochromate, pyridinium dichromate, trifluoroacetic anhydride-dimethylsulfoxide or acetic anhydride-dimethyl sulfoxide at a temperature of from −78°C to 25°C for from 5 min. to 8 hrs.

Removal of protecting groups $R^1$ and $R^2$ (15→16) is accomplished by acidic aqueous hydrolysis of 15 in a solvent such as methanol, ethanol, tetrahydrofuran or dioxane in the presence of an acid such as hydrochloric, sulfuric or acetic at a temperature of from 0 to 100°C for from 2 to 18 hours.

The diazo species 17 is prepared from 16 by treating 16 in a solvent such as $CH_3CN$, $CH_2Cl_2$ or THF with an azide such as p-carboxybenzenesulfonylazide, toluenesulfonylazide or methanesulfonylazide in the

presence of a base such as triethylamine, pyridine, $(C_2H_5)_2NH$ for from 1 to 50 hours at 0—25°C.

Cyclization (17→18) is accomplished by treating 17 in a solvent such as benzene, toluene or THF at a temperature of from 50—110°C for from 1—5 hours in the presence of a catalyst such as *bis* (acetylacetonato) Cu (II) $[Cu(acac)_2]$, $CuSO_4$, Cu powder, $Rh(OAc)_2$, or $Pd(OAc)_2$. Alternatively, the cyclization may be accomplished by irradiating 17 through a pyrex filter (a wave length greater than 300nm) in a solvent such as benzene, $CCl_4$ or diethylether at a temperature of from 0—25°C for form 0.5 to 2 hours. ["OAc" = acetate.]

Establishment of leaving group X (18→19) is accomplished by acylating the keto ester 18 with an acylating agent R°X such as p-toluenesulfonic acid anhydride, p-nitrophenylsulfonic acid anhydride, 2,4,6-triisopropylphenylsulfonic acid anhydride, methanesulfonic acid anhydride, toluenesulfonyl chloride, p-bromophenylsulfonyl chloride, wherein X is the corresponding leaving group such as toluene sulfonyloxy, p-nitrophenylsulfonyloxy, methanesulfonyloxy, p-bromophenylsulfonyloxy and other leaving groups which are established by conventional procedures and are well known in the art. Typically, the above acylation to establish leaving groups X is conducted in a solvent such as methylene chloride, acetonitrile or dimethylformamide, in the presence of a base such as diisopropylethylamine, triethylamine or 4-dimethylamino-pyridine at a temperature of from −20 to 40°C for from 0.5 to 5 hours. The leaving group X of intermediate 19 can also be halogen. The halogen leaving group is established by treating 18 with a halogenating agent such as $Ø_3PClL_2$, $ØPBr_2$, $(ØO)_3PBr_2$ or oxalyl chloride in a solvent such as $CH_2Cl_2$, $CH_3CN$ or THF, in the presence of a base such as diisopropylethylamine, triethylamine, or 4-dimethylamino-pyridine. [Ø = phenyl.]

The reaction 19→20 is accomplished by treating 19 in a solvent such as dioxane, dimethylformamide, dimethylsulfoxide, acetonitrile or hexamethylphosphoramide, in the presence of an approximately equivalent to excess of the mercaptan reagent $HSCH_2CH_2NHR^8$ wherein $R^8$ is hydrogen or a readily removable N-protecting group such as p-nitrobenzyloxycarbonyl or o-nitrobenzyloxycarbonyl, in the presence of a base such as sodium hydrogen carbonate, potassium carbonate, triethylamine, diisopropylethylamine, or the like at a temperature of from −40 to 25°C for from 1 to 72 hours. The mercaptan reagent, $HSCH_2CH_2NHR^8$, is typically prepared by treating aminoethylmercaptan in the presence of the desired acid chloride in the presence of a base such as sodium bicarbonate, sodium hydroxide, or the like in a solvent such as aqueous diethylether, aqueous dioxane, aqueous acetone, or the like at a temperature of from 0 to 25°C for form 0.5 to 4 hours.

The final deblocking step 20→I is accomplished by conventional procedures such as hydrolysis or hydrogenation. Typically 20 in a solvent such as dioxane-water-ethanol, tetrahydrofuran-aqueous dipotassium hydrogen phosphate-isopropanol or the like is treated under a hydrogen pressure of from 1 to 4 atmospheres in the presence of a hydrogenation catalyst such as palladium on charcoal, palladium hydroxide, or the like at a temperature of from 0 to 50°C for from 0.5 to 4 hours. to provide I.

In the above-discussed aldol reaction 7→8 for introduction of the hydroxyethyl side chain, the scheme proceeds directly to give a mixture of isomers (*trans*-R, *trans*-S, and *cis* R) from which the desired *trans*-R isomer can be separated chromatographically or by crystallization. An indirect aldol reaction scheme stereo-selectively provides the desired *trans*-R isomer according to the following scheme:

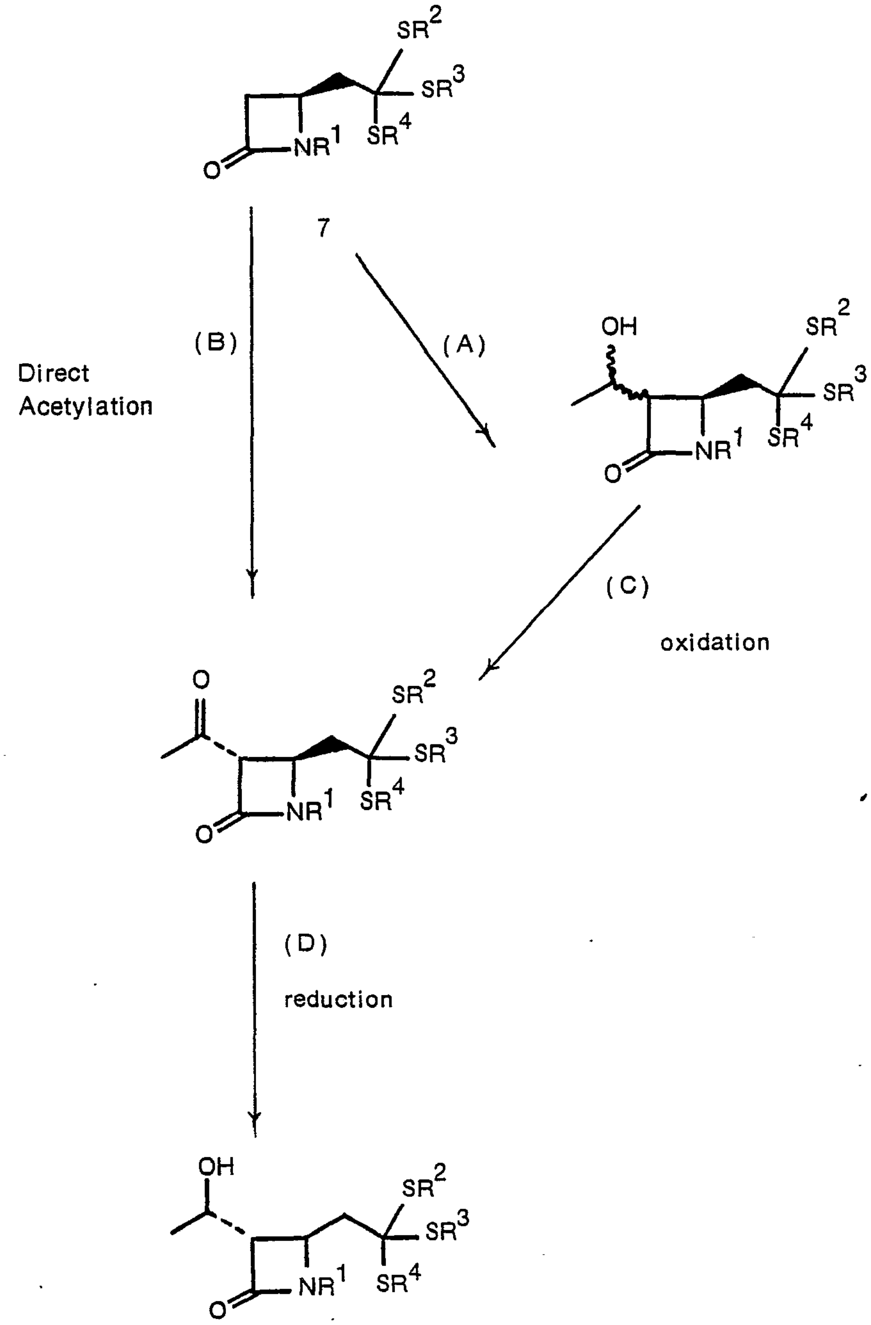


8

wherein all symbols are as previously defined.

In words relative to the above reaction scheme, Step A has been described above. The direct acetylation, Step B, is accomplished by treating 7 with two or more equivalents of a base such as lithium diisopropylamide, lithium hexamethyldisilazide, lithium 2,2,6,6-tetramethylpiperidide, in a solvent such as tetrahydrofuran, diethylether, or dimethoxyethane, for examaple, at a temperature of from −100 to −20°C with an acylating agent such as N-acetyl imidazole. Addition of the 7 plus base mixture to the acylating agent is preferred.

The oxidation Step C is accomplished with an oxidizing agent such as dipyridine chromium(VI)oxide, trifluoroacetic anhydride-dimethylsulfoxide-triethylamine, pyridinium dichromate, acetic anhydride-dimethylsulfoxide in a solvent such as methylene chloride or acetonitrile, at a temperature of from −78 to 25°C for from 5 minutes to 5 hours.

The reduction Step D is accomplished by contacting the ketone with a reducing agent such as potassium tri(sec-butyl)borohydride, lithium tri(sec.-butyl) borohydride, sodium borohydride, sodium tris-(methoxyethoxy)aluminum hydride or lithium aluminum hydride in a solvent such as diethylether, tetrahydrofuran or toluene at a temperature of from −20 to 25°C. The reaction can conveniently be conducted in the presence of an added complexing salt such as potassium iodide or magnesium bromide.

In the foregoing word description of the above schematic reaction diagram for the total synthesis of thienamycin, it is to be understood that there is considerable latitude in selection of precise reaction parameters. Suggestion of this latitude and its breadth is generally indicated by the enumeration of equivalent solvent systems, temperature ranges, protecting groups, and range of identities of involved reagents.

The following examples recite the preparation of the claimed compounds as well as their use for the total synthesis. It is to be understood that the purpose of this recitation is to further illustrate the total synthesis and not to impose any limitation.

Example 1

Preparation of 4(S)-4-Iodomethylazetidin-2-one

STEP A

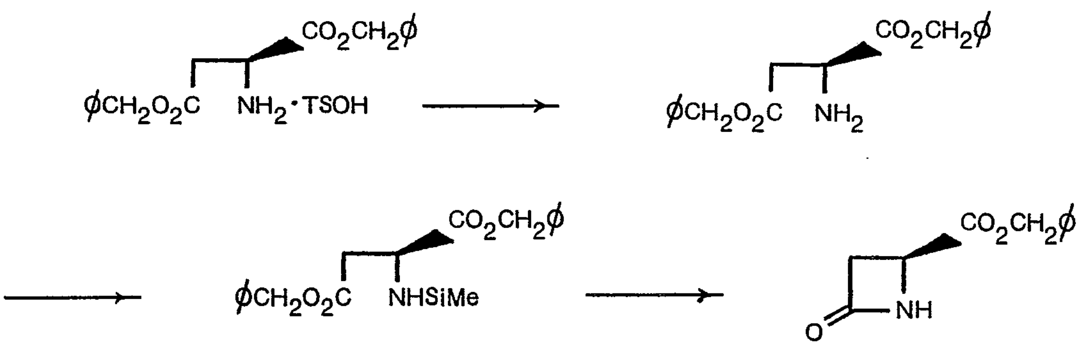

Benzyl (*S*)-azetidin-2-one-4-carboxylate

To a 1000 ml separatory funnel are added dibenzyl (*S*)-aspartate *p*-toluenesulfonic acid salt (48.6 g, 0.1 mole), ice-cold diethyl ether (300 ml), ice-cold water (100 ml), and ice-cold saturated aqueous potassium carbonate (50 ml). The mixture is shaken vigorously and the layers are separated. The aqueous portion is extracted with more cold diethyl ether (2 × 100 ml). The combined ether solution is washed with brine, dried with magnesium sulfate, and evaporated under vacuum to provide dibenzyl (*S*)-aspartate (31.4 g, 0.1 mole) as a colorless liquid.

The dibenzyl (*S*)-aspartate in anhydrous diethyl ether (200 ml) is cooled in an ice-bath under a nitrogen atmosphere. Trimethylchlorosilane (12.7 ml, 0.1 mole) is added to the stirred solution to give a white precipitate. Triethylamine (14.0 ml, 0.1 mole) is then added to the mixture. The cooling bath is removed and the mixture is stirred at room temperature (22—25°C) for 2 hrs. The mixture is then filtered directly into a 3-neck, 1.0 liter, round bottom flask fitted with a sintered glass funnel, magnetic stirrer, and a vacuum-nitrogen inlet. This operation is carried out under a blanket of nitrogen, care being taken to exclude atmospheric moisture. The sintered glass funnel is replaced by a stopper and the ether is evaporated under vacuum with stirring to provide dibenzyl (*S*)-N-trimethylsilylaspartate (35.5 g, 0.092 mole) as a slightly hazy oil.

Anhydrous diethyl ether (250 ml) is added to the flask containing the silyl derivative and the magnetic stirrer is replaced by a mechanical stirrer. The resulting solution is stirred under a nitrogen atmosphere with ice-bath cooling. Ethereal ethyl magnesium bromide (34 ml of a 2.94 M solution, 0.1 mole) is added dropwise over 40 min. to give a cream coloured, stirable precipitate. The cooling bath is removed and the mixture is stirred at room temperature. After 1.5 hrs, a viscous gum forms. The mixture is allowed to stand overnight at room temperature. The mixture is then cooled in a ice-methanol bath while ammonium chloride saturated 2N hydrochloric acid (100 ml) is added slowly with stirring. The resulting mixture is diluted with ethyl acetate (100 ml) and water (100 ml) and the layers are separated. The aqueous portion is extracted with more ethyl acetate (3 × 100 ml). The combined organic solution is washed with water (200 ml), 5% aqueous sodium bicarbonate solution (100 ml), water (100 ml), and brine, dried with magnesium sulfate, and filtered. Evaporation of the solvent under vacuum gives an orange oil interspersed with a fine, granular precipitate (25.3 g). This material is dissolved in warm chloroform (75 ml), diluted with petroleum ether (125 ml), seeded, scratched, and cooled in an ice-bath. The precipitate is collected, washed with petroleum ether, and dried under vacuum to give benzyl (*S*)-azetidin-2-one-4-carboxylate (3.85 g) as an off-white solid mp 136—139°C. The mother liquors and washings are combined, diluted with petroleum ether to 500 ml, seeded, and left in a refrigerator for several days. The resulting precipitate is collected, washed with petroleum ether, and dried under vacuum to give additional product (0.82 g) as pale yellow crystals. Recrystallization of a sample from chloroform-petroleum ether gave the product as small, white flakes: mp 141—143°; $[\alpha]_D = -43.4°$ (c3.275 in $CHCL_3$); IR($CHCl_3$) 3425, 1778, 1746 $cm^{-1}$; $^1H$ NMR($CDCl_3$) δ 3.00 (ddd, 1, J=1.9, 3.2, and 14.6 Hz, H—3a), δ 3.35 (ddd, 1, J=1.5, 5.4, and 14.6 Hz, H—3b), δ 4.20 (dd, 1, J= 3.2 and 5.4

Hz, H—4), δ 5.22 (s, 2, $OCH_2Ph$), δ 6.48 (m, 1, NH), 7.38 (s, 5, phenyl); mass spectrum m/e 205 (M+), 163, 91, 70, 43.

*Anal.* Calcd. for $C_{11}H_{11}NO_3$: C, 64.38; H, 5.40; N, 6.83. found: C, 64.10; H, 5.70; N, 6.77.

STEP B

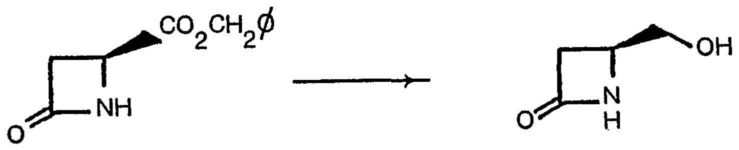

4(*S*)-4-Hydroxymethylazetidin-2-one

Sodium borohydride (3.69 g, 97.5 mmol) is added in one portion to a suspension of benzyl 4(*S*)-azetidin-2-one-4-carboxylate (20.0 g, 97.5 mmol) in 300 ml of absolute methanol at 0°C. The mixture is then allowed to warm slowly with periodic cooling being supplied to keep the internal temperature <30°C. After stirring for 2 hr., glacial acetic acid (23.4 g, 390 mmol) is added and the reaction mixture is concentrated under vacuum. The residue is treated with 500 ml of chloroform and filtered. The filtrate is concentrated under vacuum and the residue is chromatographed on 250 g of silica gel (4:1, chloroform: methanol) to yield 9.62 g (98%) of 4(*S*)-hydroxymethylazetidin-2-one as a white solid: m.p. 51—53°; $[\alpha]_D$= +68.0° (C=2.676 in $CHCL_3$); IR ($CHCl_3$) 3410, 1765 $cm^{-1}$ 1H NMR ($CDCl_3$) δ 7.07 (1H, br. s, N<u>H</u>). δ 4.05 (1H, br. s, O<u>H</u>), δ 3.77 (2H, m H4, H-5a or b), δ 3.58 (1H, dd, J=11, 6, H-5a or b), δ 2.97 (1H, ddd, J=14.5, 4.8, 1.3, H3b), δ 2.7 (1H, br. d, J=14.5, H3a); mass spectrum m/e 101 (M+), 83.

STEP C

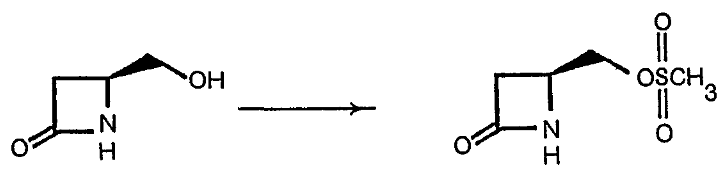

4(*S*)-4-Methanesulfonyloxymethyl azetidin-2-one

Methanesulfonyl chloride (11.46 g, 100 mmol) is added dropwise by syringe to a solution of 4(*S*)-4-hydroxymethyl azetidin-2-one (10.1 g, 100 mmol) and triethyl amine (10.1 g, 100 mmol) in 15 ml of dry methylene chloride at 0·C. (Warming is necessary in order to initially solubilize the alcohol. The resulting solution is then cooled to 0°C prior to addition of the other reagents). The resulting solution is stirred at 0°C for 1 hr. during which time a voluminous precipitate is produced. At the end of this time, the reaction mixture is filtered and the filtrate is concentrated under vacuum. The two solid residues are combined and treated with 500 ml of chloroform. The resulting mixture is filtered to yield substantially pure 4(*S*)-methanesulfonyloxymethyl azetidin-2-one as a white solid. The filtrate, which contains most of the triethylamine hydrochloride, is concentrated under vacuum and chromatographed on 200 g of silica gel (4:1 chloroform:methanol) to yield an additional quantity of mesylate. This material is combined with that obtained previously and recrystallized from chloroform to yield 15.57 g (87%) of 4(*S*)-4-methanesulfonyloxymethylazetidin-2-one as colorless needles: m.p. 109.5—110.5°C; $[\alpha]_D$= +25.8° (C=1.025 in $H_2O$); NMR ($D_2O$) δ 4.62 (1H, dd, J=11.2, 3.0, H-5a or b), δ 4.43 (1H, dd, J=11.2, 6, H-5a or b), δ 4.12 (1H, m, H4), δ 3.26

(3H, s, 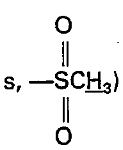)

δ 3.19 (1H, dd, J=15, 4.5, H3b).

δ 2.88 (1H, dd, J=15, 2.5, H3a); mass spectrum m/e 179 (M+), 136;

*Anal:* Calc: C, 33.51; H, 5.06; N, 7.82; S, 17.89.

Found: C, 33.54; H, 5.08; N, 7.22; S, 17.93.

STEP D

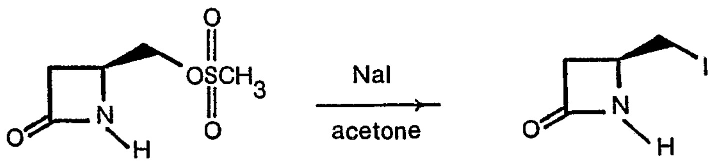


4(*S*)-4-Idomethylazetidin-2-one

A mixture of 4(*S*)-4-methanesulfonyloxy azetidin 2-one (11.8 g, 65.9 mmol) and powdered sodium iodide (19.8 g, 132 mmol) in 130 ml of acetone is heated at reflux for 6 hr. The resulting reaction mixture is concentrated *in vacuo*, treated weith 200 ml of chloroform and filtered. The filtrate is washed with 2 × 50 ml of water and dried over magnesium sulfate. The organic phase is filtered, concentrated *in vacuo*, and chromatographed on 250 g of silica gel (ethyl acetate) to yield 11.94 g (86%) of 4(*S*)-4-iodomethyl-azetidin-2-one as a white solid. This material is recrystallized from ether-petroleum ether to yield white crystals: mp 91—92°C; $[\alpha]_D$= −23.7° (C=1.354 in $CHCl_3$); IR ($CHCl_3$) 3450, 1765 $cm^{-1}$, 1H NMR ($CHCl_3$) δ 6.13 (brs, N—H), δ 3.94 (m, 1 H, Hc), δ 3.36 (m, 2H, Hd and e), δ 3.16 (ddd, 1H, J=14.9, 5.4, 2.3, Ha), δ 2.72 (d, d, d, 1H, J=14.9, 2.1, 2, Hb) mass spectrum m/e 211 ($M^+$), 168, 142, 127, 84.

Example 2

Preparation of (4*S*)-1-(t-Butyldimethylsilyl)-4-iodomethylazetidin-2-one

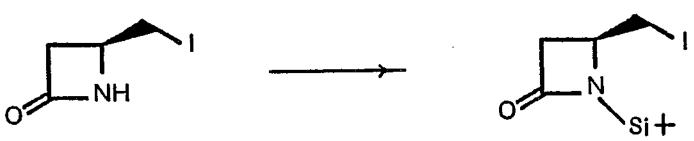

t-butyldimethylchlorosilane (7.51 g, 49.8 mmol) is added in one portion to an ice-cold, stirring solution of (4*S*)-4-iodomethyl-azetidin-2-one(10.0g, 47.4mmol) and triethylamine (5.04g, 49.8mmol) in anhydrous dimethylformamide (100 ml). A voluminous white precipitate forms almost immediately. The reaction mixture is stirred at 0—5° for 1 hour and then allowed to warm to room temperature. Most of the solvent is removed under vacuum to give a residue which is partitioned between diethyl ether (250 ml) and water. The ethereal phase is washed with 2.5N hydrochloride acid (50 ml), water (3×50 ml), and brine, dried with magnesium sulfate, filtered, and evaporated under vacuum to provide (4*S*)-1-(t-butyldimethylsilyl)-4-iodomethyl-azetidin-2-one (15.1 g) as a white solid. Recrystallization from petroleum ether — ethyl ether gives the product as colorless plates, mp 71—72°; n.m.r. ($CDCl_3$), δ 3.8 (m,l), δ 2.6—3.6 (2 overlapping d of AB, 4) δ 1.0 (S, 9) δ 0.3 (S, 6), δ 0.25 (S, 6).

Example 3

Preparation of (4S)-1-(t-Butyldimethylsilyl)-4-(2,2,2-tri(methylthio)-ethyl)azetidin-2-one

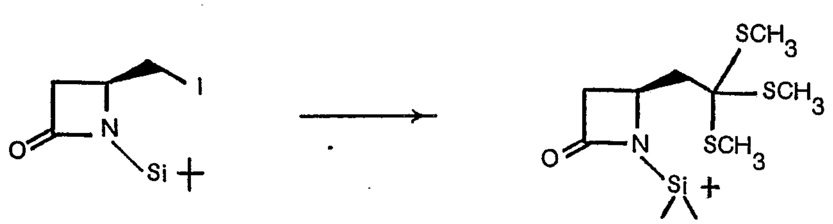

n-Butyllithium (19.4 ml of 2.5M hexane solution, 48.5 mmol) is added slowly by syringe to a solution of tri(methylthio)methane (7.47 g, 48.5 mmol) in 150 ml of freshly distilled THF at −78°C. The resulting solution is stirred at −78°C for 30 min. prior to the addition of a solution of (4*S*)-1-(tert-butyldimethylsilyl)-4-idomethylazetidin-2-one (15.0 g, 46.15 mmol) in 50 ml of THF. This solution is stirred at −78°C for 30 min., then quenched by addition of saturated aqueous ammonium chloride solution. The reaction mixture is allowed to warm to room temperature, then poured into ether (250 ml), washed with water (2×100 ml) brine (100 ml) and dried over magnesium sulfate. Removal of solvents *in vacuo* gives an oil which is crystallized from petroleum ether to give 13.3g (82%) of (4S)-1-(t-butyldimethylsilyl)-4-(2,2,2-tri(methylthio)ethyl)azetidin-2-one as colorless prisms. m.p. 61—62°C. IR($CHCl_3$, $CM^{-1}$) 2918, 2850, 1730; n.m.r. ($CDCl_3$) δ 4.0 (m, l), δ 3.35 (dd, l, J=5.5, 16), δ 2.83 (dd, l, J=3, 16) δ 2,5 (ABq, 2) δ 2, 15 (s, 9), δ 0.98 (s, 9), δ 0.25 (s, 6).

Example 4

Preparation of (3S, 4R)-1-(t-butyldimethylsilyl)-3-[(R)-1-hydroxyethyl]-4-[2,2,2-tri(methylthio)ethyl]-azetidin-2-one

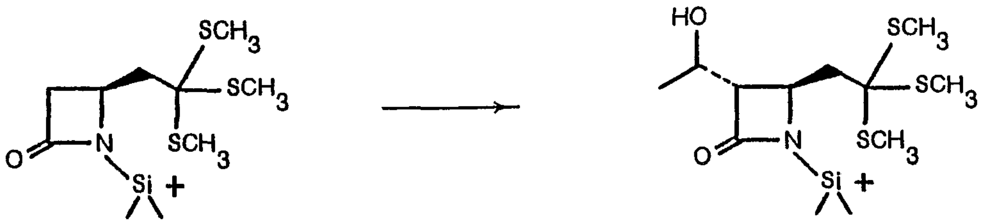

n-Butyllithium (14.8 ml of 2.5N hexane solution, 37.0 mmol) is added by syringe to a solution of diisopropylamine (3.74 g, 37.0 mmol) in 180 ml of freshly distilled tetrahydrofuran at −78°C. The resulting solution is stirred at −78°C for 15 min prior to the addition of a solution of (4S)-1-(t-butyldimethylsilyl)-4-[2,2,2-tri(methylthio)ethyl]azetidin-2-one (12.34 g, 35.16 mmol) in 35 ml of tetrahydrofuran. This solution is stirred at −78°C for 10 min prior to the addition of acetaldehyde (4.62 g, 105 mmol). The solution is stirred for an additional 5 min. at −78°C and then quenched by addition of saturated aqueous ammonium chloride solution, and allowed to warm to room temperature. The mixture is poured into 250 ml of ether and washed with water (2 × 100 ml) and brine and dried over magnesium sulfate. Removal of solutions *in vacuo* gives an oil which is chromatographed on a silica gel column (1:1 ether: petroleum ether) to give (3*S*, 4R)-1-(t-butyldimethylsilyl)-3-[(R)-1-hydroxyethyl]-4-[2,2,2-tri(methylthio)ethyl]-azetidin-2-one (7.0 g, 50.4%) at $R_f$=0.2. The product can be rcrystallized from petroleum ether. Alternatively, the trans R product can be isolated from the crude reaction mixture by direct crystallization from a petroleum ether solution.

Example 5

Preparation of (3S, 4R)-1-(t-Butyldimethylsilyl)-3-(1-oxoethyl)-4-[2,2,2,-tri(methylthio)ethyl] azetidin-2-one

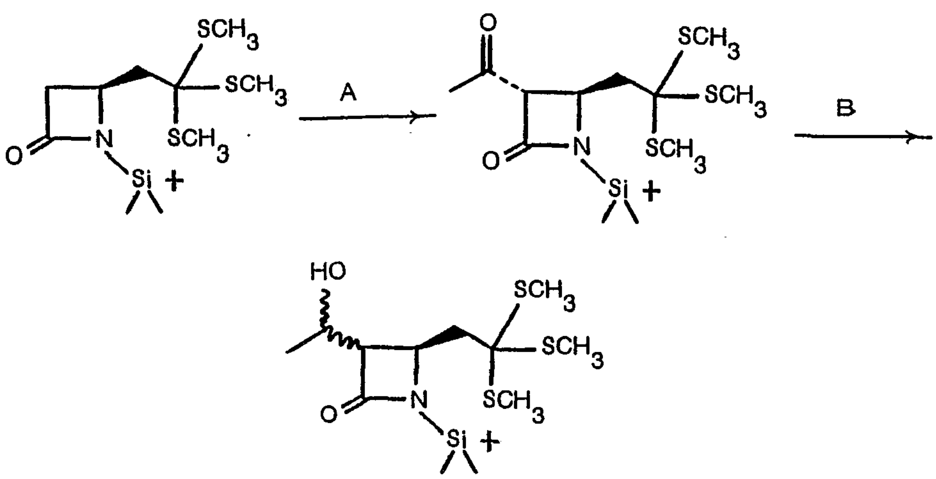


A. n-Butyllithium (2.43 ml of 2.4m solution, 5.84 mmol) is added by syringe to a solution of diisopropylamine (591 mg, 5.84 mmol) in 25 ml of freshly distilled tetrahydrofuran at −78°C. The resulting solution is stirred at −78°C for 15 minutes prior to the addition of a solution of (4*R*)-1-(t-butyldimethylsilyl)-4-[2,2,2-tri(methylthio)ethyl]azetidin-2-one (1.00 g, 2,85 mmol) in tetrahydrofuran (5 ml). This solution is stirred at −78°C for 15 minutes, then added *via* a Teflon tube to a mixture of N-acetylimidazole (642 mg, 5.84 mmol) in 25 ml of THF at −78°C. The resulting yellow reaction mixture is stirred at −78°C for 10 minutes, then quenched by addition of saturated aqueous ammonium chloride solution. The mixture is diluted with ether (200 ml) and washed with 2.5N hydrochloric acid solution (50 ml), water (50 ml) and brine (50 ml) and dried over magnesium sulfate. Removal of solvents *in vacuo* gives a yellow oil which is chromatographed on silica gel (30% ether in petroleum ether) to yield (3*S*, 4*R*)-1-(t-butyldimethylsilyl)-3-(1-oxoethyl)-4-[2,2,2-tri(methylthio)ethyl] azetidin-2-one. n.m.r. ($CDCl_3$) δ 4.42 (m, l), δ 4.32 (d, l) δ 2.35 (m, 2), δ 2.32 (s, 3), δ 2.2 (s, 9), δ 0.98 (s, 9), δ 0.3 (2s, 6).

B. Trifluoroacetic anhydride (400 mg., 1.905 mmol) is added by syringe to a solution of dimethyl sulfoxide (2.53 mmol) in dry methylene chloride (5 ml) at −78°C. The resulting mixture is stirred at −78°C for 30 minutes prior to the addition of a solution of (3RS, 4R)-1-(t-butyldimethylsilyl)-3-[(RS)-1-hydroxyethyl]-4-[2,2,2-tri(methylthio)ethyl] azetidin-2-one (500 mg., 1.27 mmol) in dry $CH_2Cl_2$ (1 ml). The resulting solution is stirred for 30 minutes prior to the addition of triethylamine (360 mg., 3.56 mmol). The cooling bath is removed. After 40 minutes, the reaction mixture is diluted with $CH_2Cl_2$, washed with water

and brine and dried over magnesium sulfate. Removal of solvents *in vacuo* gives an oil which is purified as above. Yields 432 mg. (86%).

Example 6

Preparation of (3*S*, 4*R*)-1-(t-butyldimethylsilyl)-3-[(*R*)-1-hydroxyethyl]-4-[2,2,2-tri(methylthio)ethyl] azetidin-2-one

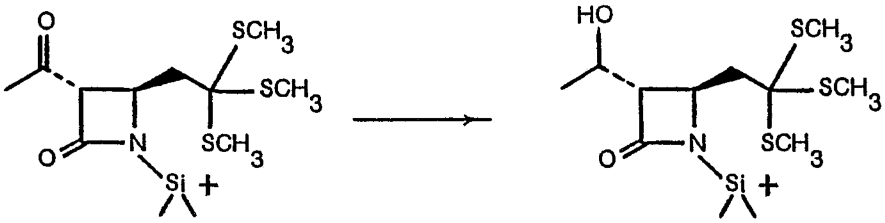

K-Selectride® (3.64 ml of 0.5M, 1.82 mmol) is added by syringe to a mixture of potassium iodide (126 mg., 0.758 mmol) and (3*S*, 4*R*)-1-(t-butyl dimethylsilyl)-3-(1-oxoethyl)-4-[2,2,2-tri(methylthio)ethyl] azetidin-2-one (298 mg, 0.758 mmol) in freshly distilled ethyl ether (8 ml) at room temperature. The resulting mixture is stirred at room temperature for 2.5 hours, then quenched by the addition of acetic acid (218 mg., 3.64 mmol). The resulting mixture is diluted with ethyl acetate (25 ml) and filtered through celite. Removal of solvents *in vacuo* gives an oil which is chromatographed on silica gel (ether:petroleum ether) to yield 252 mg of (3*S*, 4*R*)-1-(t-butyldimethylsilyl)-3-[(*R*-1-hydroxyethyl]-4-[2,2,2-tri(methylthio) ethyl] azetidin-2-one. N.M.R. (*R* isomer, $CDCl_3$+$D_2O$) δ 4.15 (dq, I), δ 3.95 (ddd, I, J=9.5, 2.3), δ 3.26 (dd, I, J=8, 2.3), δ 2.37 (m, 2), δ 2.16 (s, 9), δ 1.37 (d,3, J=6, 6), δ 1.0 (s, 9), δ 0.26 (s, 6).

Example 7

Preparation of (3R, 4R)-1-(t-Butyldimethylsilyl)-3-((R)-1-hydroxyethyl)-4-carbomethoxymethylazetidin-2-one

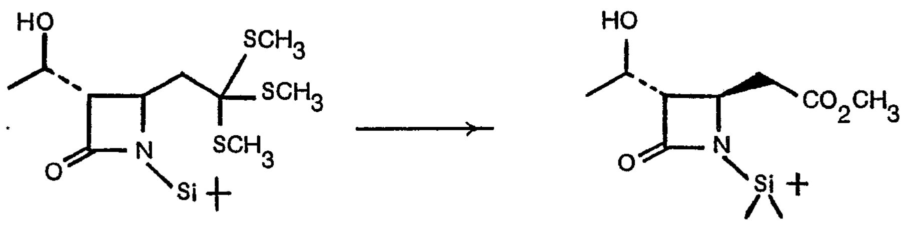

Mercuric chloride (12.37 g, 45.6 mmol) is added in one portion to a solution of (3S, 4R)-1-(t-butyldimethylsilyl)-3-[(R)-1-hydroxyethyl]-4-[2,2,2-tri(methylthio)-ethyl] azetidin-2-one (6.0 g, 15.2 mmol) in 250 ml of absolute methanol at 0°C. The resulting mixture (heavy white precipitate) is stirred at 0°C for 3 min., then quenched by addition of sodium bicarbonate (8.99 g, 107 mmol). This mixture is then filtered and the solid residue is washed with additional methanol. The combined filtrate and washings are concentrated *in vacuo* and the residue is partitioned between ethyl acetate and saturated aqueous ammonium chloride solution. The organic phase is separated, washed with saturated aqueous ammonium chloride solution, water and brine and dried over magnesium sulfate. Removal of solvents *in vacuo* gives an oil which is chromatographed on a silica gel column (3:2 cyclohexane:ethyl acetate) to yield (3S, 4R)-1-(t-butyldimethylsilyl)-3-[(R)-1-hydroxyethyl)]-4-carbomethoxymethyl azetidin-2-one.

Example 8

Preparation of (3S, 4R)-1-(t-Butyldimethylsilyl)-3-[(R)-1-(t-butyldimethylsilyloxy)ethyl]-4-carbomethoxymethylazetidin-2-one

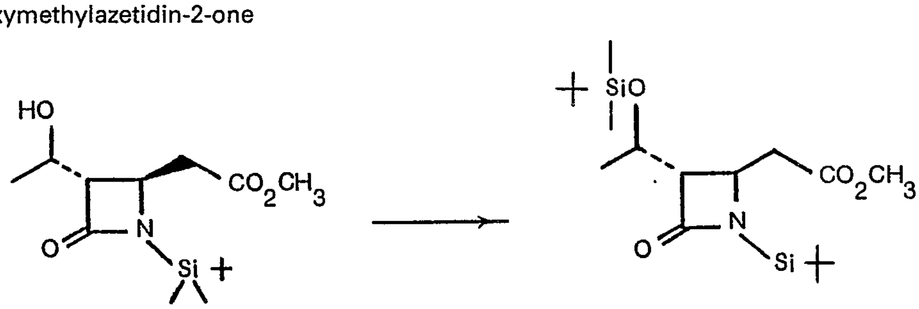

t-Butyldimethylchlorosilane (940 mg, 6.25 mmol) is added in one portion to a solution of (3S, 4R)-1-(t-butyldimethylsilyl)-3-[(R)-1-hydroxyethyl]-4-carbomethoxymethylazetidin-2-one (1.88 g, 6.25 mmol) and triethylamine (1.27 g, 6.25 mmol) in 15 ml of anhydrous dimethylformamide at 0°C. After 15 min. at 0°C the

cooling bath is removed and the reaction mixture is stirred at room temperature for 24 hrs. Ether (100 ml) is added and the mixture is filtered, then washed with 2.5N hydrochloric acid (20 ml), water (3×20 ml) and brine. The organic phase is dried over magnesium sulfate, then concentrated *in vacuo*. The residue is chromatographed on silica gel (7:3 petroleum ether:ether) to yield (3S, 4R)-1-(t-butyldimethylsilyl)-3-[(R)-1-(t-butyldimethylsilyloxy)ethyl]-4-carbomethoxymethylazetidin-2-one. n.m.r. ($CDCl_3$) δ 4.1 (m, 2), δ 3.68 (S, 3), δ 3.03 (dd, l, J=4.3, 2.7), δ 2.8 (ABq, 2), δ 1.17 (d, 3, J=6.6), δ 0.98 (s, 9), δ 0.89 (s, 9), δ 0.23 (s, 6), δ 0.1 (s, 6).

Example 9

Preparation of (3S, 4R)-1-(t-Butyldimethylsilyl)-3-[(R)-1-(t-butyldimethylsilyloxy)ethyl]-4-(2-oxoethyl)azetidin-2-one

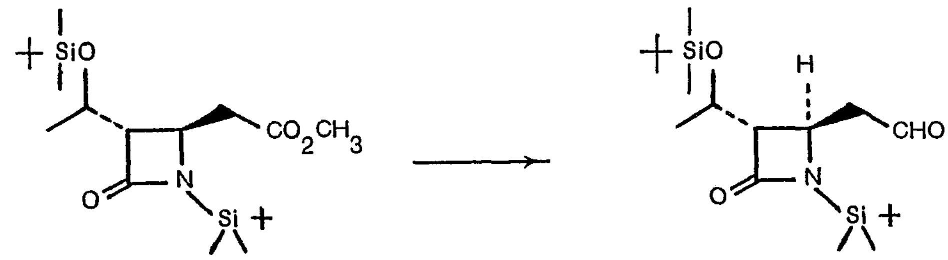

Diisobutylaluminum hydride (3.72 ml of 0.91 M in hexane, 3.38 mmol) is added slowly by syringe to a solution of (3S, 4R)-1-(t-butyldimethylsilyl)-3-[(R)-1-(t-butyldimethylsilyloxy)ethyl]-4-carbomethoxymethyl azetidin-2-one (936 mg, 2.26 mmol) in 25 ml of freshly distilled toluene at −78°C. The resulting solution is stirred at −78°C for 3 hrs., then quenched by addition of 2.5N hydrochloric acid (5 ml). The resulting mixture is stirred for 2 min., then poured into a separatory funnel containing 100 ml of ether and 50 ml of 1.25N hydrochlroic acid saturated with tartaric acid. The organic phase is separated and the aqueous phase is extracted with ether (2×50 ml). The combined organic phases are washed with brine and dried over magnesium sulfate. Removal of solvents *in vacuo* gives a white solid which is recrystallized from ether-petroleum ether to give (3S, 4R)-1-(t-butyldimethylsilyl)-3-[(R)-1-(t-butyldimethylsilyloxy)ethyl]-4-(2-oxoethyl)-azetidin-2-one. m.p. 115—116°C; n.m.r. ($CDCl_3$) δ 4.1 (m, l), δ 4.03 (m, l), δ 2.7—3.2 (m, 3), δ 1.23 (d, 3, J=6.4), δ 1.08 (s, 9), δ 0.9 (s, 9), δ 0.25 (s, 6), δ 0.1 (s, 6), δ 9.83 (t, l, J=1.4).

Example 10

Preparation of . (3S, 4R)-1-(t-Butyldimethylsilyl)-3-[(R)-1-(t-butyldimethylsilyloxy)ethyl]-4-(3-benzyloxycarbonyl-2-hydroxypropyl)azetidin-2-one

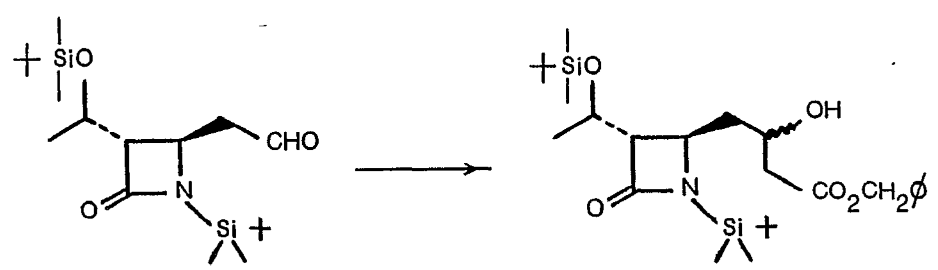

n-Butyllithium (1.81 mmol) is added by syringe to a solution of diisopropylamine (1.81 mmol) in 9 ml of freshly distilled tetrahydrofuran at −78°C. The resulting solution is stirred for 15 min at −78°C. Benzyl acetate (1.81 mmol) is then added dropwise by syringe and the resulting solution is stirred at −78°C for 20 min. A solution of (3S, 4R)-1-(t-butyldimethylsilyl)-3-[(R)-1-(t-butyldimethylsilyloxy)ethyl]-4-(2-oxoethyl)azetidin-2-one (1.64 mmol) in 3 ml of anhydrous tetrahydrofuran is added slowly by syringe. The reaction mixture is stirred at −78°C an additional 15 min and then quenched by addition of saturated aqueous ammonium chloride solution. Ethyl acetate (50 ml) is added and the organic phase is separated, washed with water (2×10 ml) and brine and dried over magnesium sulfate. Removal of solvents *in vacuo* gives a white solid which is chromatographed on a short silica gel column (40% ether in petroleum ether) to yield (3S, 4R)-1-(t-butyldimethylsilyl)-3-[(R)-1-(t-butyldimethylsilyloxy)ethyl]-4-(3-benzyloxycarbonyl-2-hydroxypropyl)-azetidin-2-one. n.m.r. ($CDCl_3$) δ 7.32 (s, 5), δ 5, 1, (s, 2), δ 4.0 (m, 3), δ 2, 4—3, 8 (m, 4), δ 2.0 (m, 2), δ 1.25 (overlapping d, 3), δ 0.95 (s, 9), δ 0.9 (s, 9), δ 0.3 (s, 6), δ 0.18 (s, 6).

Example 11

Preparation of (3S, 4R)-1-(t-Butyldimethylsilyl)-3-[(R)-1-(t-butyldimethylsilyloxy)ethyl]-4-[3-(4-nitrobenzyl)oxycarbonyl-2-hydroxypropyl]azetidin-2-one

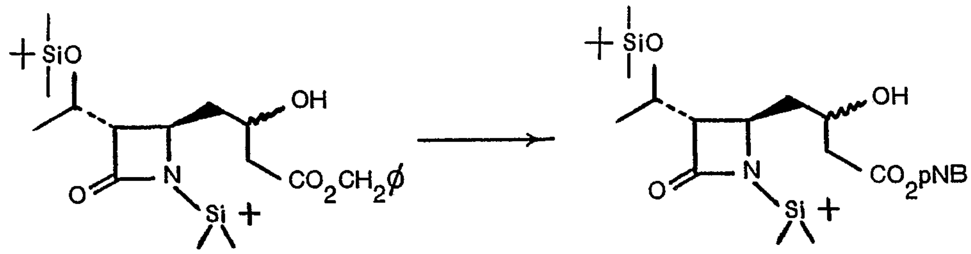

A mixture of (3S, 4R)-1-(t-butyldimethylsilyl)-3-[(R)-1-(t-butyldimethylsilyloxy)ethyl]-4-(3-benzyloxycarbonyl-2-hydroxypropyl)azetidin-2-one (1.00 mmol), sodium bicarbonate (1.00 mmol) and 10% Pd/C in 20 ml of 4:1 tetrahydrofuran—$H_2O$ is hydrogenated at 40 psi on a Parr shaker for 30 min. The mixture is then filtered through Celite and the catalyst is washed with 10 ml of water. The combined washings and filtrate are concentrated i.v. to 2 ml and lyophilized. The resulting fluffy white solid is dissolved in 5 ml of anhydrous dimethylformamide and p-nitrobenzyl bromide (216 mg, 1.00 mmol) is added in one portion. The resulting solution is stirred at room temeprature for 3 hrs, then diluted with ether (50 ml) and washed with water (3×10 ml) and brine and dried over magnesium sulfate. The solvents are removed *in vacuo* and the residue is chromatographed on silica gel to yield (3S, 4R)-1-(t-butyldimethylsilyl)3[(R)-1-(t-butyldimethylsilyloxy)ethyl]-4-[3-(4-nitrobenzyl)oxycarbonyl-2-hydroxypropyl]azetidin-2-one. n.m.r. ($CDCl_3$) δ 7.85 (2d-aromatic, 4), δ 5, 26 (s, 2), δ 4.2 (m, 3), δ 2, 5—3.6 (m, 4) δ 2.0 (m, 2), δ 1.4 (2 overlapping d, 3), δ 1.0 (2s, 18), δ 0.25 (2s, 12).

Example 12

Preparation of (3S, 4R)-1-(t-Butyldimethylsilyl)-3-[(R)-1-(t-butyldimethylsilyloxy)ethyl]-4-[3-(4-nitrobenzyl)oxycarbonyl-2-oxopropyl]azetidin-2-one

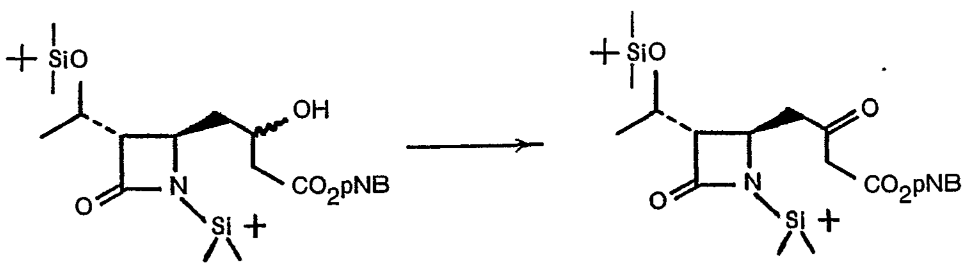

Anhydrous chromium trioxide (10.0 mmol) is added to a solution of anhydrous pyridine (20.0 mmol) in 30 ml of anhydrous methylene chloride. After stirring at room temperature for 15 min., the reaction mixture is treated all at once with a solution of (3S, 4R)-1-(t-butyldimethylsilyl)-3-[(R)-1-(t-butyldimethylsilyloxy) ethyl -4-[3-(4-nitrobenzyl) oxycarbonyl-2-hydroxypropyl]-azetidin-2-one (1.00 mmol) in anhydrous methylene chloride (8 ml). The resulting mixture is stirred at room temperature for 5 min. The $CH_2Cl_2$ layer is decanted from the dark, tarry residue which is triturated with more $CH_2Cl_2$. The combined $CH_2Cl_2$ phase is concentrated *in vacuo*. The residue is triturated with ether (100 ml) and the ether extracts are filtered. The filtrate is washed with 5% aqueous sodium bicarbonate solution, 2.5N HCl, 5% $NaHCO_3$ and brine, dried over magnesium sulfate and concentrated *in vacuo* to yield (3S, 4R)-1-(t-butyldimethylsilyl)-3-[(R)-1-(t-butyldimethylsilyloxy)ethyl]-4-[3-(4-nitrobenzyl)oxycarbonyl-2-oxopropyl]azetidin-2-one. n.m.r. ($CDCl_3$) δ 7.85 (2d-aromatic, 4), δ 5.27 (s, 2), δ 4.05 (m, 2), δ 3.6 (s, 2), δ 2.4—3.2 (dd overlapping ABq, 3), δ 1.2 (d, 3, J=6.6), δ 0.9 (2s, 18), δ 0.22 (s, 6), δ 0.05 (s, 6).

Example 13

Preparation of (3S, 4R)-3-[(R)1-hydroxyethyl]-4-[3-(4-nitrobenzyl)oxycarbonyl-2-oxopropyl]azetidin-2-one

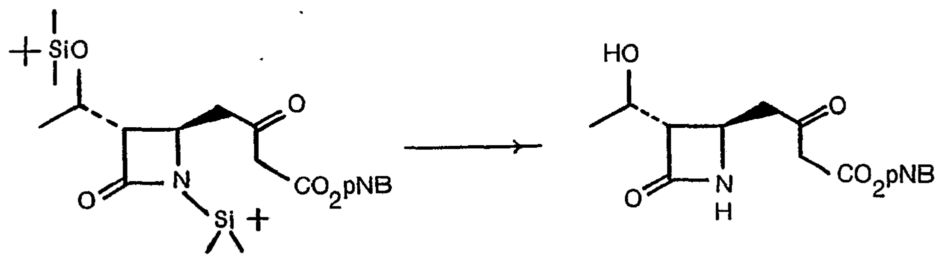

(3S, 4R)-1-(t-butyldimethylsilyl)3-[(R)-1-(t-butyldimethylsilyloxy)ethyl]-4-[3-(4-nitrobenzyl)oxycarbonyl-2-oxopropyl]azetidin-2-one (7.9 mmol) is dissolved in 160 ml of 9:1 (v/v) methanol-water and cooled to 0°C. Concentrated hydrochloric acid (2.75 ml) is added and the resulting solution is stirred at 0°C for 15 min., then allowed to warm to room temperature. The solution is stirred at room temperature for 2.5 hrs, then diluted with ethyl acetate (200 ml) and washed with water, saturate aqueuous sodium bicarbonate solution and brine, dried over magnesium sulfate and concentrated *in vacuo* to yield (3S, 4R)-3-[(R)1-hydroxyethyl]-4-[3-(4-nitrobenzyl)oxycarbonyl-2-oxopropyl]-azetidin-2-one.

Example 14

Preparation of (3*S*, 4*R*)-3-[(R)-1-Hydroxyethyl]-4-[3-(4-nitrobenzyl)-oxycarbonyl-2-oxo-3-diazopropyl]-azetidin-2-one

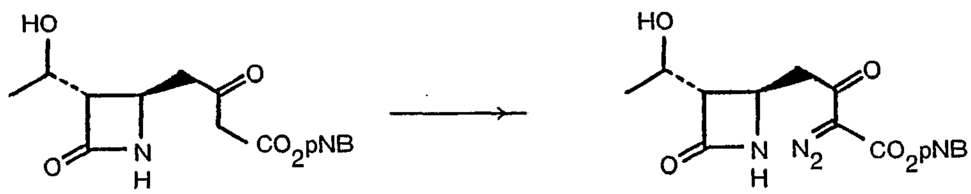

Triethylamine (263 mg, 2.6 mmol) is added by syringe to a mixture of (3*S*, 4*R*)-3-[(R)-1-hydroxyethyl]-4-[3-(4-nitrobenzyl)oxycarbonyl-2-oxopropyl]azetidin-2-one (253 mg, [0.72] mmol) and p-carboxybenzene sulfonylazide (196 mg, 0.84 mmol) in dry acetonitrile (6 ml) at 0°C. When addition is complete the cooling bath is removed and the reaction mixture is stirred at room temperature for 1 hour. The mixture is then diluted with ethyl acetate (50 ml) and filtered. The filtrate is concentrated *in vacuo* and the residue is chromatographed on a short silica gel column (ethyl acetate) to yield 222 mg, (81% overall from (3*S*, 4*R*)-1-(t - butyldimethylsilyl) - 3 - [(R) - 1 - (t - butyl dimethylsilyloxy)ethyl] - 4 - [3 - (4 - nitrobenzyl)oxycarbonyl - 2-oxopropyl]azetidin-2-one) of (3*S*, 4*R*)-3-(R)-(1-hydroxyethyl)-4-[3-(4-nitrobenzyl)oxycarbonyl-2-oxo-3-diazopropyl]azetidin-2-one as a white solid m.p. (dec.) 163°C. IR($CHCl_3$, $CM^{-1}$) 3410, 2132, 1756, 1718, 1650, 1350, 1280, 1120; n.m.r. ($CDCl_3$) δ 7.9 (2d-aromatic, 4), δ 5.4 (s, 2), δ 6.2 (brs, 1), δ 4.1 (m, 2), δ 2.6—3.6 (m, 4), δ 1.32 (d, 3, J=6.2).

Example 15

Preparation of (5*R*, 6*S*)p-Nitrobenzyl 6-[(*R*)1-hydroxyethyl]-1-azabicyclo[3.2.0]heptan-3,7-dione-2-carboxylate

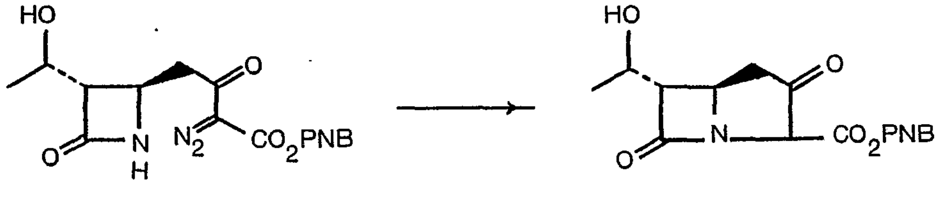

A suspension of (3*S*, 4*R*)-3-[(R)-1-hydroxyethyl]-4-[3-(4-nitrobenzyl)oxycarbonyl-2-oxo-3-diazopropyl]azetidin-2-one (56.4 mg, 0.15 mmol) and rhodium (II) acetate (0.1 mg) in dry benzene (3 ml) is deoxygenated by bubbling through nitrogen for 10 minutes. The mixture is then heated to +78°C for 1 hour. During heating the solid starting material gradually goes into solution. The mixture is then cooled, filtered to remove the catalyst, and the filtrate is concentrated *in vacuo* to yield (5*R*, 6*S*) p-nitrobenzyl 6-[(R)-1-hydroxyethyl]-1-azabicyclo[3.2.0]heptan-3,7-dione-2-carboxylate, 51 mg. (98%) as a colorless oil which slowly crystallized at room temperature (22°C).

Physical Properties:

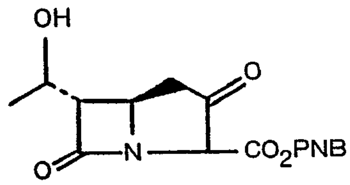

PNB = p-nitrobenzyl

n.m.r.: (300MHz, $CDCl_3$) δ 8.26, 7.54 (aromatic, 4), 5.29 (AB, 2), 4.77 (s, 1), 4.32 (dq, 1, J=6.6, 7), 4.16 (ddd, 1, J=7, 7.5, 2.2), 3.21 (dd, 1, J=7, 2.2), 2.94 (dd, 1, J=19.5, 7) 2.50 (dd, 1, J=19.5, 7.5), 2.2 (brs, 1), 1.37 (d, 3, J=6.6).
I.R.: ($CHCl_3$, $CM^{-1}$) 1770, 1758, 1610, 1522, 1353
m.p. 110—111°C.

The following examples show the use of the present compounds for the preparation of thienomycin.

Example 16

Preparation of *p*-Nitrobenzyloxycarbonylaminoethanethiol

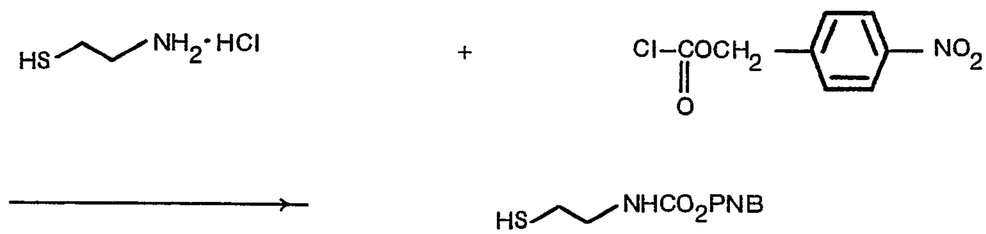

To 600 ml diethyl ether ($Et_2O$) — 75 ml $H_2O$ in an ice bath with stirring is added 3.2 g cysteamine hydrochloride (mw = 114; 28.1 mmole). A solution of 7.14 g $NaHCO_3$ (mw = 84; 85 mmole) in 75 ml $H_2O$ is added. The ice bath is removed, and at room temperature a solution of 6.75 g *p*-nitrobenzylchloroformate (mw = 216; 31.3 mmole) in 270 ml $Et_2O$ is added dropwise over a period of one hour. After 10 additional minutes, the layers are separated. The ether layer is extracted with 150 ml 0.25 N HCl, and then with 200 ml brine. Each aqueous layer is then backwashed successively with 100 ml $Et_2O$. The combined $Et_2O$ layers are dried over anhydrous $MgSO_4$, filtered, and concentrated under a $N_2$ stream. The crystalline residue is slurried in a small amount of ether, filtered, and the pale yellow crystals are dried under high vacuum to give 4.7 g. *p*-nitrobenzyloxycarbonylaminoethanethiol (65% yield). NMR ($CDCl_3$): 8.18 (*d*, J=8Hz, aroatic protons ortho to nitro), 7.47 (*d*, J=8Hz, aromatic protons meta to nitro), 5.27 (—N<u>H</u>—), 5.20 (*s*, _C<u>H</u>$_2$—NH—), 2.67 (*m*, —C<u>H</u>$_2$—SH), 1.35 (*t*, J=8.5Hz, —S<u>H</u>)in ppm downfield from TMS. IR ($CHCl_3$ solution): carbonyl— 1725 $cm^{-1}$. M.S.: molecular ion—256, (M—47) at 209, (M—136) at 120, $^+CH_2\O pNO_2$ at 136.

Example 17

Preparation of (5*R*, 6*S*) p-Nitrobenzyl 3-[2-(p-nitrobenzyloxy-carbonyl)amino ethylthio]-6-[(R)-1-hydroxyethyl]-1-azabicyclo [3, 2, 0]-hept-2-en-7-one-2-carboxylate

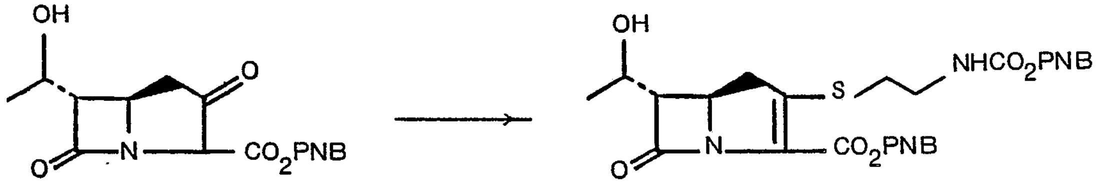

(5*R*, 5*S*) p-Nitrobenzyl 6-[(*R*)1-hydroxyethyl]-1-azabicyclo [3, 2, 0]heptan-3,7-dione-2-carboxylate (51 mg, 0.147 mmol) is dissolved in acetonitrile (3 ml) and the resulting solution is cooled to 0°C. Diisopropylethylamine (22 mg, 0.17 mmol) is added by syringe and the resulting solution is stirred at 0°C for 1 minute prior to the addition of a solution of freshly recrystallized p-toluene sulfonic anhydride (51 mg., 0.156 mmol) in dry acetonitrile (1 ml). The resulting solution containing the isolable inermediate (5*R*, 6*S*) p-Nitrobenzyl-3-(p-toluene sulfonyloxy)-6-[(R)-1-hydroxyethyl]-1-azabicyclo[3.2.0]hept-2-en-7-one-2-carboxylate is stirred at 0°C for 1 hour, then cooled to −25°C. Dissiopropylethylamine (80.5 mg, 0.624

mmol) is added by syringe followed shortly thereafter by a solution of N-p-nitrobenzyloxycarbonyl-cysteamine (40 mg, 0.156 mmol) in 1 ml of dry acetonitrile. The reaction mixture is then stored in a refrigerator for 70 hr. The mixture is diluted with 25 ml of ethyl acetate washed with brine and dried over magnesium sulfate. Solvents are removed *in vacuo* to yield a yellow oil which is chromatographed on a silica gel plate (ethyl acetate, $R_f$=0.4) to yield (5*R*, 6*S*) p-nitrobenzyl-3-[2-(p-nitrobenzyloxycarbonyl)amino ethylthio]-6-[(R)-1-hydroxyethyl]-1-azabicyclo [3,2,0]-hept-2-en-7-one-2-carboxylate as a yellow solid, m.p. 167—169°C. IR(Nujol mull) 1773 and 1690 $cm^{-1}$; n.m.r. ($CDCl_3$) δ 7.54—8.26 (overlapping ABq, 4), δ 5.40 (ABq, 2), δ 5.22 (s, 2), δ 4.27 (m, 2), δ 3.47 (m), δ 3.23 (dd, 1), δ 3.14 (dd, 1), δ 3.40 (dd, 1), δ 3.04 (m, 2), δ 1.37 (d, 3).

Example 18

Preparation of Thienamycin

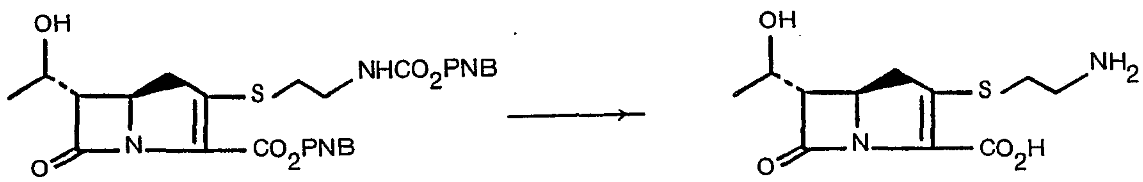

A mixture of N-p-nitrobenzyloxycarbonyl thienamycin p-nitrobenzyl ester (10 mg, 0.017 mmol) and 10% Pd/C-Bolhofer type in tetrahydrofuran (2 ml), 0.1M dipotassium hydrogen phosphate solution (1.4 ml) and 2-propanol (0.2 ml) is hydrogenated at 40 psi on the Parr shaker for 30 minutes. The mixture is then filtered and the catalyst is washed with water (3 × 3 ml). The combined filtrate and washings are extracted with ethyl acetate-ethyl ether then concentrated to ~3 ml and lyophilized. The resulting white powder is identical to natural thienamycin in all respects.

**Claims for the Contracting States: BE CH DE FR GB IT LU NL SE**

1. The compound:

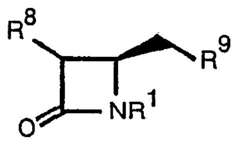

wherein

a) $R^1$ is hydrogen or a protecting group,
   $R^8$ is hydrogen and
   $R^9$ is iodo,

b) $R^1$ is a protecting group,
   $R^8$ is

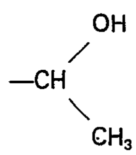

$R^9$ and is $—C(SR)_3$
wherein R is selected from alkyl having 1—6 carbon atoms, aryl and aralkyl having 6 to 10 carbon atoms, and wherein R can be the same or different,

c) $R^1$ is hydrogen, $R^8$ is

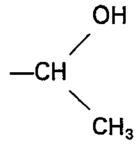

and
$R_9$ is

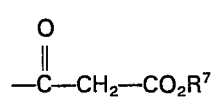

wherein $R^7$ is a protecting group,

d) $R^1$ is hydrogen,
$R^8$ is

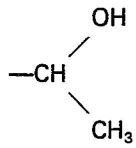

and
$R^9$ is

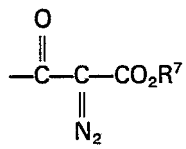

wherein $R^7$ is a protecting group.

2. The compound:

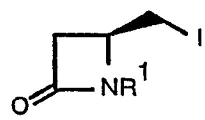

wherein $R^1$ is hydrogen or a protecting group.

3. The compound:

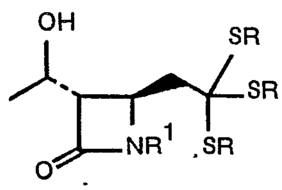

wherein $R^1$ is a protecting group and R is selected from alkyl having 1—6 carbon atoms, aryl and aralkyl having 6 to 10 carbon atoms and R can be the same or different.

4. The compound:

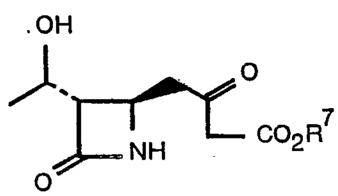

wherein $R^7$ is a protecting group.

5. The compound.

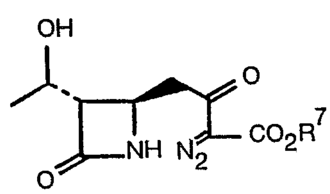

wherein $R^7$ is a protecting group.

6. A process for preparing a compound according to Claim 2 which comprises treatment of a compound

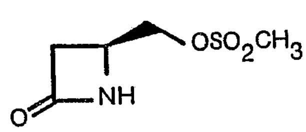

with sodium iodide and inserting a protecting group if desired.

7. A process for preparing a compound according to Claim 3 which comprises alkylation of a compound

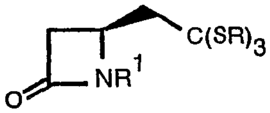

wherein R and $R^1$ are as defined in claim 3, with acetaldehyde in a solvent with a strong base.

8. A process for preparing a compound according to Claim 4 which comprises removal of the protecting groups $R^1$ and $R^2$ of a compound

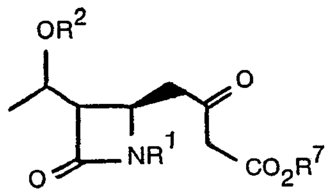

wherein $R^7$ is as defined in claim 4, by acidic hydrolysis in a solvent.

9. A process for preparing a compound according to Claim 5 which comprises treatment of a compound

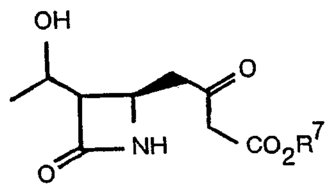

wherein $R^7$ is as defined in claim 5, in a solvent with an azide in the presence of a base.

**Claims for the Contracting State: AT**

1. A process for preparing a compound of the formula

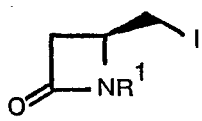

wherein $R^1$ is hydrogen or a protecting group which comprises treatment of a compound

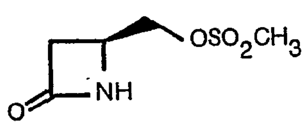

with sodium iodide and inserting a protecting group if desired.

2. A process for preparing a compound of the formula

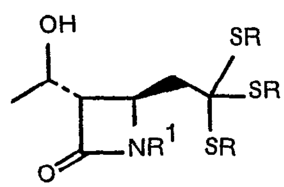

wherein $R^1$ is a protecting group and R is selected from alkyl having 1—6 carbon atoms, aryl and aralkyl having 6 to 10 carbon atoms and R can be the same or different which comprises alkylation of a compound

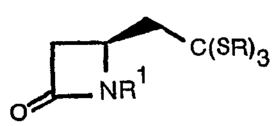

wherein R and $R^1$ are as defined above, with acetaldehyde in a solvent with a strong base.

3. A process for preparing a compound of the formula

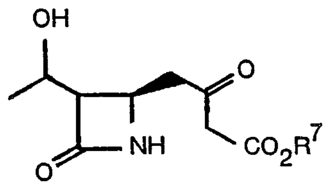

wherein $R^7$ is a protecting group which comprises removal of the protecting groups $R^1$ and $R^2$ of a compound

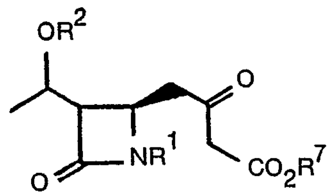

wherein $R^7$ is as defined above, by acidic hydrolysis in a solvent.

4. A process for preparing a compound of the formula

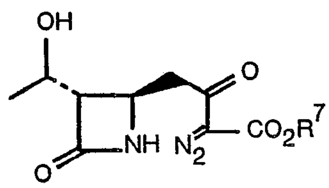

wherein $R^7$ is a protecting group which comprises treatment of a compound

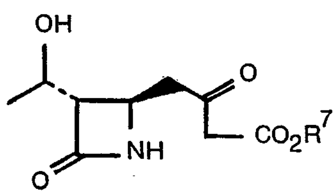

wherein $R^7$ is as defined above, in a solvent with an azide in the presence of a base.

**Patentansprüche für die Vertragsstaaten: BE CH DE FR GB IT LU NL SE**

1. Die Verbindung:

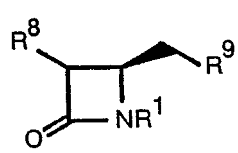

worin

a) $R^1$ Wasserstoff oder eine Schutzgruppe ist,
$R^8$ Wasserstoff ist und
$R^9$ Iod ist,

b $R^1$ eine Schutzgruppe ist,
$R^8$

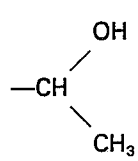

ist und
$R^9$ $—C(SR)_3$ ist, wobei R aus Alkyl mit 1—6 Kohlenstoffatomen, Aryl sowie Aralkyl mit 6—10 Kohlenstoffatomen ausgewählt ist, und wobei die Reste R gleich oder verschieden sein können,

c) $R^1$ Wasserstoff ist,
$R^8$

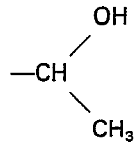

ist und
$R^9$

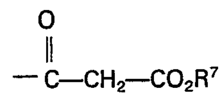

ist, wobei $R^7$ eine Schutzgruppe ist,

d) $R^1$ Wasserstoff ist,
$R^8$

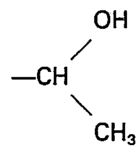

ist und
$R^9$

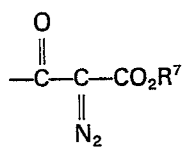

ist, wobei $R^7$ eine Schutzgruppe ist.

2. Die Verbindung:

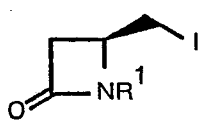

worin $R^1$ Wasserstoff oder eine Schutzgruppe ist.

3. Die Verbindung:

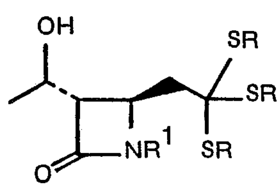

worin $R^1$ eine Schutzgruppe ist und R aus Alkyl mit 1—6 Kohlenstoffatomen, Aryl sowie Aralkyl mit 6 bis 10 Kohlenstoffatomen ausgewählt ist, und die Reste R gleich oder verschieden sein können.

4. Die Verbindung:

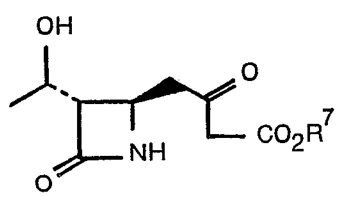

worin $R^7$ eine Schutzgruppe ist.

5. Die Verbindung:

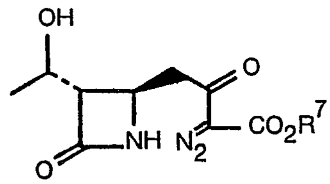

worin $R^7$ eine Schutzgruppe ist.

6. Ein Verfahren zur Herstellung einer Verbindung nach Anspruch 2, welches die Behandlung einer Verbindung

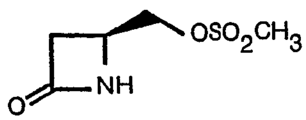

mit Natriumiodid und gewünschtenfalls die Einführung einer Schutzgruppe umfaßt.

7. Ein Verfahren zur Herstellung einer Verbindung nach Anspruch 3, welches die Alkylierung einer Verbindung

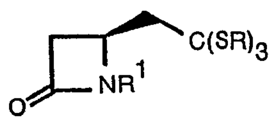

worin R und $R^1$ wie im Anspruch 3 definiert sind, mit Acetaldehyd in einem Lösungsmittel mit einer starken Base umfaßt.

8. Ein Verfahren zur Herstellung einer Verbindung nach Anspruch 4, welches die Entfernung der Schutzgruppen $R^1$ und $R^2$ aus einer Verbindung

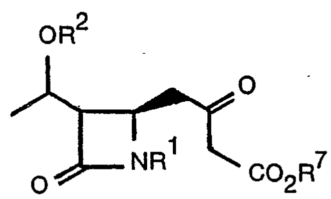

worin $R^7$ wie im Anspruch 4 definiert ist, durch saure Hydrolyse in einem Lösungsmittel umfaßt.

9. Ein Verfahren zur Herstellung einer Verbindung nach Anspruch 5, welches die Behandlung einer Verbindung

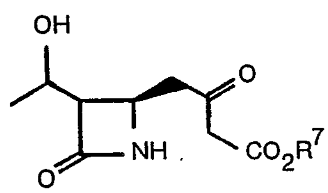

worin $R^7$ wie im Anspruch 5 definiert ist, in einem Lösungsmittel mit einem Azid in Gegenwart einer Base umfaßt.

**Patentansprüche für den Vertragsstaat: AT**

1. Ein Verfahren zur Herstellung einer Verbindung der Formel

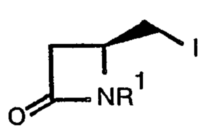

worin $R^1$ Wasserstoff oder eine Schutzgruppe ist, welches die Behandlung einer Verbindung

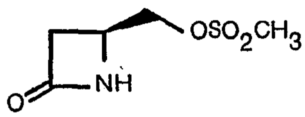

mit Natriumiodid und gewünschtenfalls die Einführung einer Schutzgruppe umfaßt.

2. Ein Verfahren zur Herstellung einer Verbindung der Formel

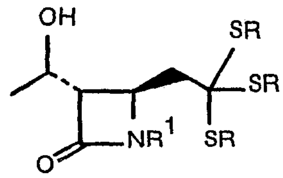

worin $R^1$ eine Schutzgruppe ist und R aus Alkyl mit 1—6 Kohlenstoffatomen, Aryl sowie Aralkyl mit 6 bis 10 Kohlenstoffatomen ausgewählt ist, und die Reste R gleich oder verschieden sein können, welches die Alkylierung einer Verbindung

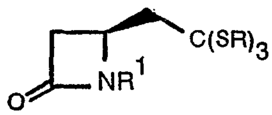

worin R und $R^1$ wie oben definiert sind, mit Acetaldehyd in einem Lösungsmittel mit einer starken Base umfaßt.

3. Ein Verfahren zur Herstellung einer Verbindung der Formel

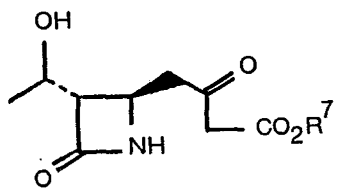

worin $R^7$ eine Schutzgruppe ist, welches die Entfernung der Schutzgruppen $R^1$ und $R^2$ aus einer Verbindung

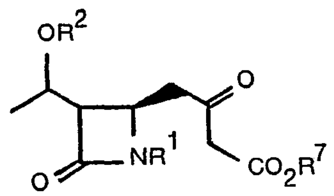

worin $R^7$ wie oben definiert ist, durch saure Hydrolyse in einem Lösungsmittel umfaßt.

4. Ein Verfahren zur Herstellung einer Verbindung der Formel

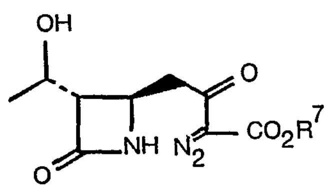

worin $R^7$ eine Schutzgruppe ist, welches die Behandlung einer Verbindung

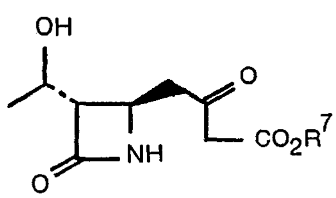

worin $R^7$ wie oben definiert ist, in einem Lösungsmittel mit einem Azid in Gegenwart einer Base umfaßt.

**Revendications pour les Etats contractants: BE CH DE FR GB IT LU NL SE**

1. Le composé:

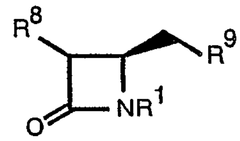

où

a) $R^1$ est un hydrogène ou un groupe protecteur, $R^8$ est un hydrogène et $R^9$ est un iodo,

b) $R^1$ est un groupe protecteur, $R^8$ est

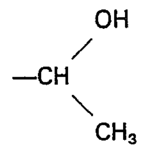

$R^9$ est $-C(SR)_3$ où R est choisi parmi un alkyle ayant 1 à 6 atomes de carbone, un aryle et un aralkyle ayant 6 à 10 atomes de carbone, et où les R peuvent être semblables ou différents,

c) $R^1$ est un hydrogène, $R^8$ est

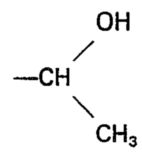

et $R^9$ est

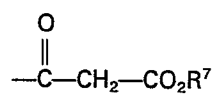

où $r^7$ est un groupe protecteur,

d) $R^1$ est un hydrogène, $R^8$ est

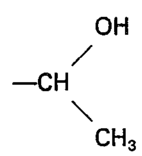

et $R^9$ est

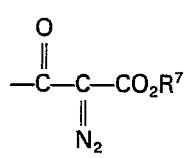

où $R^7$ est un groupe protecteur.

2. Le composé:

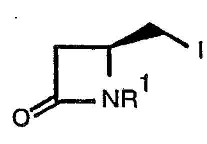

où $R^1$ est un hydrogène ou un groupe protecteur.

3. Le composé:

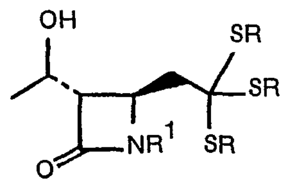

où $R^1$ est un groupe protecteur et R est choisi parmi un alkyle ayant 1 à 6 atomes de carbone, un aryle et un aralkyle ayant 6 à 10 atomes de carbone et les R peuvent être semblables ou différents.

4. Le composé:

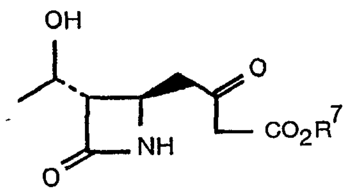

où $R^7$ est un groupe protecteur.

5. Le composé:

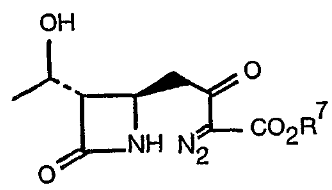

où $R^7$ est un groupe protecteur.

6. Un procédé pour la préparation d'un composé selon la revendication 2 qui comprend le traitement d'un composé

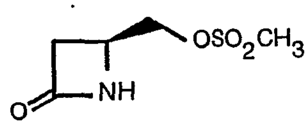

avec l'iodure de sodium et l'insertion si on le désire d'un groupe protecteur.

7. Un procédé pour la préparation d'un composé selon la revendication 3 qui comprend l'alkylation d'un composé

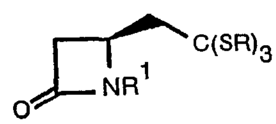

où R et $R^1$ sont comme défini dans la revendication 3, avec l'acétaldéhyde dans un solvant avec une base forte.

8. Un procédé pour la préparation d'un composé selon la revendication 4 qui comprend l'élimination des groupes protecteurs $R^1$ et $R^2$ d'un composé

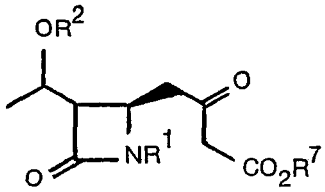

où $R^7$ est comme défini dans la revendication 4, par hydrolyse acide dans un solvant.

9. Un procédé pour la préparation d'un composé selon la revendication 5 qui comprend le traitement d'un composé

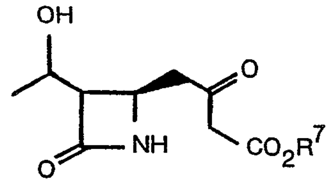

où $R^7$ est comme défini dans la revendication 5, dans un solvant avec un azide en présence d'une base.

**Revendications pour l'Etat contractant: AT**

1. Un procédé pour la préparation d'un composé de formule

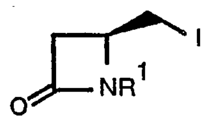

où $R^1$ est un hydrogène ou un groupe protecteur qui comprend le traitement d'un composé

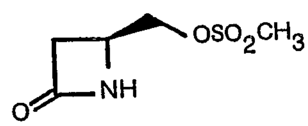

avec de l'iodure de sodium et si on le désire l'insertion d'un groupe protecteur.

2. Un procédé pour la préparation d'un composé de formule

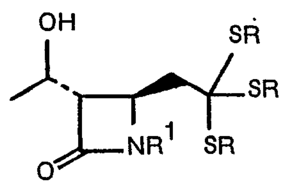

où $R^1$ est un groupe protecteur et R est choisi parmi un alkyle ayant 1 à 6 atomes de carbone, un aryle et un aralkyle ayant 6 à 10 atomes de carbone et les R peuvent être semblables ou différents, qui comprend l'alkylation d'un composé

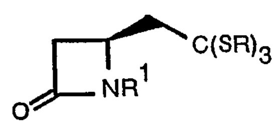

où R et $R^1$ sont comme défini ci-dessus, avec l'acétaldéhyde dans un solvant avec une base forte.

3. Un procédé pour la préparation d'un composé de formule

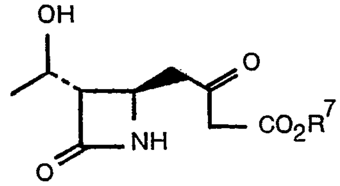

où $R^7$ est un groupe protecteur, qui comprend l'élimination des groupes protecteurs $R^1$ et $R^2$ d'un composé

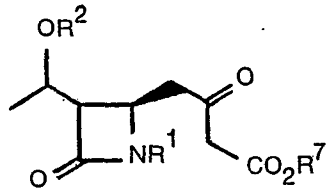

où $R^7$ est comme défini ci-dessus, par hydrolyse acide dans un solvant.

4. Un procédé pour la préparation d'un composé de formule

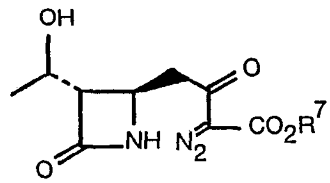

où $R^7$ est un groupe protecteur qui comprend le traitement d'un composé

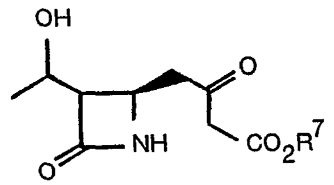

où $R^7$ est comme défini ci-dessus, dans un solvant avec un azide en présence d'une base.